# EUROPEAN PATENT APPLICATION

(11) **EP 2 762 876 A1**
(43) Date of publication of application: **06.08.2014**
(21) Application number: 12835058.4
(22) Date of filing: 26.09.2012
(51) Int. Cl.: G01N 30/88, G01N 30/32, G01N 30/56

(54) **METHOD FOR MEASURING STABLE HEMOGLOBIN A1c USING LIQUID CHROMATOGRAPHY, AND METHOD FOR SIMULTANEOUS MEASUREMENT OF STABLE HEMOGLOBIN A1c AND ABNORMAL HEMOGLOBIN USING LIQUID CHROMATOGRAPHY**

(30) Priority: 29.09.2011 JP 2011214993; 29.09.2011 JP 2011214994
(71) Applicant: Sekisui Medical Co., Ltd., Tokyo 103-0027 (JP)
(72) Inventor: OISHI, Kazuyuki, Ryugasaki-shi, Ibaraki 301-0852 (JP)
(74) Representative: Hart-Davis, Jason
(86) International application number: PCT/JP2012/074652
(87) International publication number: WO 2013/047563

(57) **Abstract**

An object of the present invention is to provide a method capable of measuring stable hemoglobin A1c using liquid chromatography in a short time with good reproducibility.

The present invention is a method for measuring stable hemoglobin A1c using liquid chromatography, installing on a flow path of a liquid chromatograph a filter whose surface is treated with a solution containing 1 to 50% by weight of a silicone oil or a solution containing 1 to 50% by weight of a silicone resin,; and setting a pressure value generated in a measurement system of the liquid chromatograph to 9.8 × 10³ Pa or more and 19.6 × 10⁵ Pa or less.

## Description

### TECHNICAL FIELD

The present invention relates to a method for measuring stable hemoglobin A1c using liquid chromatography. The present invention also relates to a method for simultaneous measurement of stable hemoglobin A1c and abnormal hemoglobin using liquid chromatography.

### BACKGROUND ART

Measurement of hemoglobins using liquid chromatography can measure stable hemoglobin A1c in a short time with high accuracy, and therefore is used in particular for control of a hemoglobin A1c value of a diabetes patient. In this use, accuracy within about 1% or less of a CV value (%) of the hemoglobin A1c value in a reproducibility test is required.

A general approach to measuring stable hemoglobin A1c using liquid chromatography in a short time with good reproducibility is to make a column packing material particle small and uniform. However, when the column packing material particle is made small, a pressure value generated in the measurement system becomes high. For example, Patent Literature 1 discloses a method for measuring hemoglobins using liquid chromatography with a column packing material particle having an average particle diameter of 3 to 4 µm. However, the pressure value in the measurement system using the technique in Patent Literature 1 becomes as high as 5 MPa or more, and therefore a liquid chromatograph with high pressure resistance is required. Moreover, in the case where the liquid chromatograph is used in such a high pressure state for a long period of time, it is liable to cause liquid leak, shortening of column lifetime, or the like due to deterioration of a connection section in piping.

Furthermore, the pressure value in the measurement system fluctuates also by clogging of a filter used in the liquid chromatograph. The filter is usually installed in a flow path of the liquid chromatograph and removes contaminants entered in the flow path from a measurement sample or reagent or the like. For example, Patent Literature 2 discloses a column for liquid chromatography in which a fiber sintered filter obtained by stacking and sintering a stainless steel fiber is installed as a frit in an end fitting of a column. Although the stainless steel sintered filer is the most general filter for liquid chromatography, in the measurement of a biological sample such as blood, protein or the like in the blood is adsorbed on the surface of the filter, and therefore the pressure value in the measurement system is liable to rise. The rise of the pressure value in the measurement system due to the clogging of the filter is liable to cause a problem similar to the problem in the above-described measurement at high pressure using the packing material having a small particle diameter. Moreover, since the pressure value in the measurement system fluctuates and causes deformation in a chromatogram by nonspecific adsorption on the filter, the measurement reproducibility is liable to be deteriorated.

As a method for suppressing the nonspecific adsorption on the filter, for example, a technique for coating treatment of a filter with silicones is disclosed in Patent Literature 3. Patent Literature 3 states that, according to the treatment technique, the recovery rate of hemoglobin is high in a wide pH range, the nonspecific adsorption is small, and the stable hemoglobin A1c value is not adversely affected. However, the coating treatment with silicones has such defects that the clogging is liable to occur due to the increased thickness of the coating layer depending on the coating condition, that continuous use for a long period of time can lead to the rise of the pressure, and that the separation performance becomes deteriorated in the case where the measuring time is shortened. These defects become particularly prominent when such a treatment is used for the high-pressure measurement system such as the measurement system described in Patent Literature 1.

Moreover, in the simultaneous measurement of stable hemoglobin A1c and abnormal hemoglobin, the number of peaks to be separated is increased, and therefore measured values greatly fluctuate due to the deterioration in separation performance.

### CITATION LIST

### - Patent Literature

Patent Literature 1: JP 05-005730 A
Patent Literature 2: JP 07-260763 A
Patent Literature 3: JP 2000-131302 A

### SUMMARY OF INVENTION

### - Technical Problem

The present invention provides a method capable of measuring stable hemoglobin A1c using liquid chromatography in a short time with good reproducibility. Moreover, the present invention provides a method capable of simultaneously measuring stable hemoglobin A1c and abnormal hemoglobin in a short time with good reproducibility.

### - Solution to Problem

The present inventor has found that, in the measurement of hemoglobins using liquid chromatography, the measurement of stable hemoglobin A1c and the simultaneous measurement of stable hemoglobin A1c and abnormal hemoglobin can be carried out in a short time with good reproducibility by treating at least any one of a filter and a column main body installed on a flow path of a liquid chromatograph with a solution containing a fixed concentration range of a silicone oil or a solution containing a fixed concentration range of a silicone resin and carrying out measurement at a pressure within a fixed range.

The first invention of the present invention is a method for measuring stable hemoglobin A1c using liquid chromatography, comprising installing on a flow path of a liquid chromatograph a filter whose surface is treated with a solution containing 1 to 50% by weight of a silicone oil or a solution containing 1 to 50% by weight of a silicone resin; and setting a pressure value generated in a measurement system of the liquid chromatograph to 9.8 × 10³ Pa or more and 19.6 × 10⁵ Pa or less.

The second invention of the present invention is a method for measuring stable hemoglobin A1c using liquid chromatography, comprising installing on a flow path of a liquid chromatograph a column main body whose inner surface is treated with a solution containing 1 to 50% by weight of a silicone oil or a solution containing 1 to 50% by weight of a silicone resin; and setting a pressure value generated in a measurement system to 9.8 × 10³ Pa or more and 19.6 × 10⁵ Pa or less.

The third invention of the present invention is a method for measuring stable hemoglobin A1c using liquid chromatography, comprising installing on a flow path of a liquid chromatograph a filter whose surface is treated with a solution containing 1 to 50% by weight of a silicone oil or a solution containing 1 to 50% by weight of a silicone resin, and a column main body whose inner surface is treated with a solution containing 1 to 50% by weight of a silicone oil or a solution containing 1 to 50% by weight of a silicone resin; and setting a pressure value generated in a measurement system to 9.8 × 10³ Pa or more and 19.6 × 10⁵ Pa or less.

The fourth invention of the present invention is a method for simultaneous measurement of stable hemoglobin A1c and abnormal hemoglobin using liquid chromatography, comprising installing on a flow path of a liquid chromatograph a filter whose surface is treated with a solution containing 1 to 50% by weight of a silicone oil or a solution containing 1 to 50% by weight of a silicone resin; and setting a pressure value generated in a measurement system of the liquid chromatograph to 9.8 × 10³ Pa or more and 19.6 × 10⁵ Pa or less.

The fifth invention of the present invention is a method for simultaneous measurement of stable hemoglobin A1c and abnormal hemoglobin using liquid chromatography, comprising installing on a flow path of a liquid chromatograph a column main body whose inner surface is treated with a solution containing 1 to 50% by weight of a silicone oil or a solution containing 1 to 50% by weight of a silicone resin; and setting a pressure value generated in a measurement system of the liquid chromatograph to 9.8 × 10³ Pa or more and 19.6 × 10⁵ Pa or less.

The sixth invention of the present invention is a method for simultaneous measurement of stable hemoglobin A1c and abnormal hemoglobin using liquid chromatography, comprising installing on a flow path of a liquid chromatograph a filter whose surface is treated with a solution containing 1 to 50% by weight of a silicone oil or a solution containing 1 to 50% by weight of a silicone resin, and a column main body whose inner surface is treated with a solution containing 1 to 50% by weight of a silicone oil or a solution containing 1 to 50% by weight of a silicone resin; and setting a pressure value generated in a measurement system of the liquid chromatograph to 9.8 × 10³ Pa or more and 19.6 × 10⁵ Pa or less.

Hereinafter, the inventions will be described in detail, beginning with the first invention and the fourth invention.

In the method for measuring stable hemoglobin A1c according to the first invention and the method for simultaneous measurement of stable hemoglobin A1c and abnormal hemoglobin of the fourth invention, a filter whose surface is treated with a solution containing 1 to 50% by weight of a silicone oil or a solution containing 1 to 50% by weight of a silicone resin is installed on a flow path of a liquid chromatograph.

Herein, the silicone oil and the silicone resin together are also simply referred to as "silicone." Moreover, the solution containing a silicone oil and the solution containing a silicone resin together are also referred to as "silicone solution." Furthermore, the filter before the treatment with the silicone solution is applied is simply referred to as "filter," the filter after the treatment with the silicone solution is applied is referred to as "silicone-treated filter," and when both of the filters are meant, both of the filters are referred to as "filters."

Publicly known materials of the filter such as metals, resins, and glass used for liquid chromatography can be used for the filter used in the first invention and the fourth invention.

Specifically, for example, metals such as aluminum, copper, titanium, nickel, iron, chromium, and tin, alloy such as stainless steel, resins such as nylon resins, fluororesins, cellulose resins, polysulfone resins, polyether sulfone resins, polypropylene resins, polyethylene resins, acrylic resins, polyester resins, vinylon resins, polycarbonate resins, polyurethane resins, polyvinyl chloride resins, polyether ether ketone resins, epoxy resins, phenol resins, and Noryl resins, ceramics such as zirconia, and glass such as borosilicate glass and quartz glass are preferable as the material of the filter.

It is preferable that the shape of the filter is a disk body, a columnar body, a cone body, or the like in which a filtering surface facing to a direction to which a liquid reagent such as an eluent flows is circular.

A convex portion or a concave portion may be provided on the surface of the filter.

In the case where the filtering surface of the filter is circular, the preferable lower limit of the diameter is 0.5 mm and the preferable upper limit is 20 mm. When the diameter of the filter is less than 0.5 mm, the filter becomes liable to be clogged. When the diameter of the filter exceeds 20 mm, the measurement sample or the mobile phase is diffused in the filter and the separation performance may be deteriorated. More preferable lower limit of the diameter of the filter is 1 mm, and more preferable upper limit is 10 mm.

The preferable lower limit of the thickness of the filter is 0.1 mm, and the preferable upper limit is 10 mm. When the thickness of the filter is less than 0.1 mm, the filter becomes liable to be clogged. When the thickness of the filter exceeds 10 mm, the measurement sample or the mobile phase is diffused in the filter and the separation performance may be deteriorated. More preferable lower limit of the thickness of the filter is 0.2 mm, and more preferable upper limit is 5 mm.

The preferable lower limit of the pore diameter of the filter is 0.1 µm and the preferable upper limit is 20 µm. When the pore diameter of the filter is less than 0.1 µm, the filter becomes liable to be clogged. When the pore diameter of the filter exceeds 20 µm, the filtering accuracy may be lowered. More preferable lower limit of the pore diameter of the filter is 1 µm, and more preferable upper limit is 15 µm.

Moreover, as disclosed in JP 2-262054 A, a filter having, within the filter, a portion in which the pore diameter is different from that in the other portion may be used.

As the filter used in the first invention and the fourth invention, a filter processed by a publicly known method can be used. Specifically, examples of the filter include a filter obtained by processing the above-described material in a thin film form such as a fiber form, a film form, a sheet form, and a filter paper form; a filter obtained by processing a fiber comprising the above-described material in a nonwoven fabric form, a woven fabric form, or a knitted work form; a filter obtained by stacking a fiber or a powder comprising the above-described material and processing the fiber or the powder by a method such as compression; and a filter obtained by stacking and sintering a fiber or a powder comprising the above-described material.

The silicone-treated filter used in the first invention and the fourth invention is a filter whose surface is treated with the silicone solution.

The "surface of the filter" includes the outer surface and the inner surface of the filter with which the measurement sample contacts.

The silicone solution is composed of silicone and a solvent dissolving the silicone.

It is preferable that the silicone contained in the silicone solution is a silicone forming a film by curing reaction, more preferably a silicone cured by forming a crosslinked structure through condensation reaction or addition reaction.

It is preferable that, silicone oils or silicone resins containing a silicone compound such as alkyl silicone compounds such as dimethyl silicone, methyl phenyl silicone, and methyl hydrogen silicone, and modified silicone compounds in which a part of alkyl groups in alkyl silicones is substituted by an amino group, an ether group, an epoxy group, a diol group, a carboxyl group, a methacrylic group, or fluorine are preferable as the silicone.

Examples of the commercially available silicone include AY series, BY series, DY series, SE series, SF series, SH series, SR series, FZ series, and 800 RESIN series manufactured by Dow Corning Toray Silicone Co., Ltd., KC series, KE series, KF series, KM series, KR series, KS series, X series, and ES series manufactured by Shin-Etsu Chemical Co., Ltd., DMS series and PDS series manufactured by Chisso Corporation, MS series manufactured by Mitsubishi Chemical Corporation, GR series manufactured by Showa Denko K.K., TSF series and TSR series manufactured by Momentive Performance Materials Japan LLC., and BYK series manufactured by BYK Japan K.K. These silicones may be used alone or in combinations of two or more.

The solvent for dissolving the silicone is not particularly limited as long as the solvent is a liquid that dissolves the silicone to be used, however organic solvents are preferable.

Examples of the organic solvent include alcohols such as methyl alcohol, ethyl alcohol, propyl alcohol, isopropyl alcohol, and butyl alcohol, straight chain or branched chain hydrocarbons such as pentane, hexane, and octane, and aromatic hydrocarbons such as benzene, toluene, and xylene.

The lower limit of the silicone concentration in the silicone solution is 1% by weight, and the upper limit is 50% by weight. When the silicone concentration is less than 1% by weight, the treatment with the silicone becomes insufficient. When the silicone concentration exceeds 50% by weight, the filter is clogged due to the high concentration silicone. The preferable lower limit of the silicone concentration is 2% by weight, and the preferable upper limit is 45% by weight.

It is preferable that the treatment of the filter with the silicone solution is a treatment of covering the filter surface by the silicone in the silicone solution, more preferably a treatment comprising a step of bringing the filter into contact with the silicone solution and a step of covering the filter surface by the silicone in the silicone solution brought into contact with the filter.

Examples of the method for bringing the filter into contact with the silicone solution include a method of immersing the filter into the silicone solution, a method of passing the silicone solution through the filter, and a method of coating the filter with the silicone solution.

It is preferable that the method of covering the filter surface by the silicone in the silicone solution is a method of curing the silicone by causing crosslinking reaction such as condensation reaction and addition reaction on the surface of the filter by a method such as irradiation with an energy ray such as an ultraviolet ray and addition of an organic solvent under room temperature or heating after bringing the filter into contact with the silicone solution.

A publicly known additive such as a crosslinking agent may be added in bringing the filter into contact with the silicone solution or curing the silicone on the surface of the filter.

Moreover, blocking treatment may be applied to the silicone-treated filter used in the first invention and the fourth invention with a blocking reagent.

Examples of the blocking reagent include proteins such as bovine serum albumin, casein, skim milk, gelatin, hemoglobin, myoglobin, globulin, and lactoferrin, polar lipids such as phospholipid, and surfactants such as sodium dodecyl sulfate and polyethylene glycol mono-4-octylphenyl ether (Triton X-100).

The blocking treatment can be applied by, for example, a method of immersing the silicone-treated filter into the solution of the blocking reagent, a method of passing the solution of the blocking reagent through the silicone-treated filter, and a method of coating the silicone-treated filter with the solution of the blocking reagent.

The first invention is a method for measuring stable hemoglobin A1c using liquid chromatography, comprising installing the above-described silicone-treated filter on a flow path of a liquid chromatograph. Moreover, the fourth invention is a method for simultaneous measurement of stable hemoglobin A1c and abnormal hemoglobin using liquid chromatography, comprising installing the silicone-treated filter on a flow path of a liquid chromatograph.

The silicone-treated filter is installed on the flow path of the liquid chromatograph. Particularly, it is preferable that the silicone-treated filter is installed on the flow path through which a measurement sample passes. It is preferable that the silicone-treated filter is used as a filter used in publicly known usages such as a line filter installed in the middle of the flow path piping, a pre-filter installed just before the separation column, a frit installed so as to be in contact with the packing material in the end fitting of the column. Above all, the usage as a pre-filter or a frit is preferred.

In addition, in the case where the silicone-treated filter is used as a frit, the silicone-treated filter may be installed in the end fitting or the silicone treatment may be applied to the end fitting including the filter after the filter is installed in the end fitting.

In the first invention and the fourth invention, the pressure value generated in the measurement system is set to 9.8 × 10³ Pa or more and 19.6 × 10⁵ Pa or less.

In the present description, the "pressure value generated in the measurement system (hereinafter, simply referred to as pressure value)" means a pressure value generated from the whole flow path after a liquid feeding pump including a column in the flow path of a liquid chromatograph. The pressure value is a value that can be measured by, for example, a pressure gauge connected between the liquid feeding pump and the column. Moreover, the pressure range of "9.8 × 10³ Pa or more and 19.6 × 10⁵ Pa or less" is referred to as the "specified pressure value" of the present invention.

In the case where the pressure value is set to less than 9.8 × 10³ Pa, the reproducibility of the measured value for hemoglobins is deteriorated and the measuring time becomes long. Moreover, since the set pressure value is very small, it becomes difficult to stabilize the pressure value. In the case where the pressure value is set to a value exceeding 19.6 × 10⁵ Pa, the reproducibility of the measured value is deteriorated.

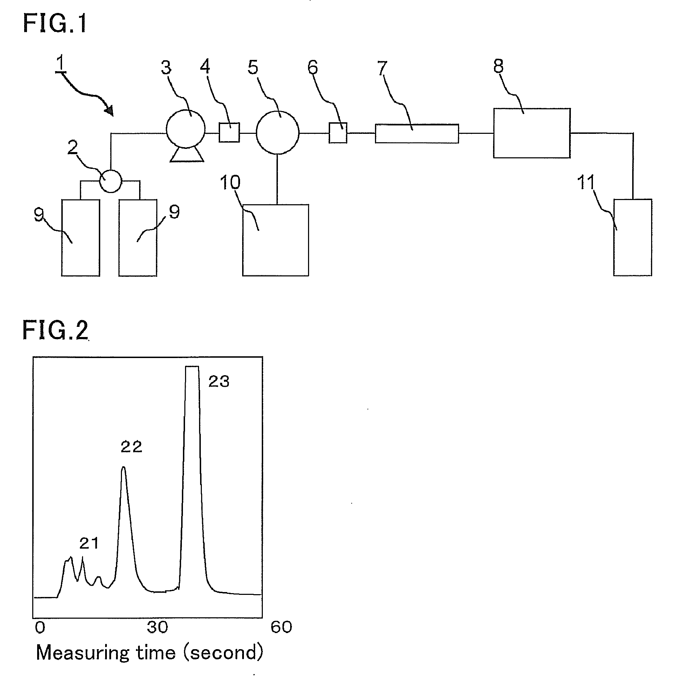

The liquid chromatography according to the first invention and the fourth invention is carried out installing the above-described silicone-treated filter in a publicly known liquid chromatograph comprising a pump for feeding liquid, a separation column, a detector, a sample introduction mechanism, and so on. A configuration example of the liquid chromatograph is illustrated in Fig. 1.

It is preferable to use a buffer solution or an organic solvent containing a publicly known salt compound as the eluent for the liquid chromatography. Examples of the buffer solution include buffer solutions of organic acids, inorganic acids, and salts thereof, and amino acids, and Good's buffer.

Examples of the organic acids include citric acid, succinic acid, tartaric acid, and malic acid.

Examples of the inorganic acids include hydrochloric acid, nitric acid, sulfuric acid, phosphoric acid, boric acid, and acetic acid.

Examples of the amino acids include glycine, taurine, and arginine.

Moreover, the substances generally added to a buffer solution such as, for example, a surfactant, various polymers, a hydrophilic low molecular weight compound, and a chaotropic compound may be appropriately added to the buffer solution in addition to those described above.

The preferable lower limit of the salt concentration of the buffer solution in carrying out the measurement of hemoglobin is 10 mmol/L, and the preferable upper limit is 1000 mmol/L. In the case where the salt concentration of the buffer solution is less than 10 mmol/L, sufficient ion exchange is not carried out and it may become difficult to separate hemoglobin. In the case where the salt concentration in the buffer solution exceeds 1000 mmol/L, it may occur that the salt in the buffer solution precipitates to adversely affect the liquid chromatograph.

Stable hemoglobin A1c can be measured in a short time with good reproducibility by the first invention. In the first invention, other hemoglobins contained in healthy human blood such as, for example, hemoglobin A0, hemoglobin F (fetal hemoglobin), and hemoglobin A2 can be measured in a short time with good reproducibility simultaneously with the measurement of stable hemoglobin A1c.

Moreover, one or more hemoglobins generally called as abnormal hemoglobin such as, for example, hemoglobin S, hemoglobin C, hemoglobin D, and hemoglobin E can be measured simultaneously with the measurement of stable hemoglobin A1c by the fourth invention.

In addition, "simultaneously" here means that the peaks of stable hemoglobin A1c and other hemoglobins are displayed on a chromatogram and the content ratio of each hemoglobin to be measured is shown in one measurement.

In the fourth invention, other hemoglobins contained in the healthy human blood such as, for example, hemoglobin A0, hemoglobin F (fetal hemoglobin), and hemoglobin A2 in addition to stable hemoglobin A1c can be measured simultaneously with abnormal hemoglobin in a short time with good reproducibility.

The second invention of the present invention is a method for measuring stable hemoglobin A1c using liquid chromatography, comprising installing on a flow path of a liquid chromatograph a column main body whose inner surface is treated with a silicone solution; and setting a pressure value generated in a measurement system of the liquid chromatograph to 9.8 × 10³ Pa or more and 19.6 × 10⁵ Pa or less.

Moreover, the fifth invention of the present invention is a method for simultaneous measurement of stable hemoglobin A1c and abnormal hemoglobin using liquid chromatography, comprising installing on a flow path of a liquid chromatograph a column main body whose inner surface is treated with a silicone solution; and setting a pressure value generated in a measurement system of the liquid chromatograph to 9.8 × 10³ Pa or more and 19.6 × 10⁵ Pa or less.

In the present description, the "column main body" designates a cylindrical part excluding an end fitting part in a column generally used for liquid chromatography, the part accommodating a column packing material. Moreover, in the present description, the column including the column main body before the silicone treatment is applied is simply referred to as "column," the column including the column main body after the treatment with the silicone solution is applied is referred to as "silicone-treated column," and when the both columns are meant, the both columns are referred to as "columns."

Publicly known materials of the column main body such as metals, resins, and glass used for a liquid chromatograph can be used as the column main body used in the second invention and the fifth invention.

Specifically, for example, metals such as aluminum and titanium, alloy such as stainless steel, resins such as nylon resins, fluororesins, cellulose resins, polysulfone resins, polyether sulfone resins, polypropylene resins, polyethylene resins, acrylic resins, polyester resins, vinylon resins, polycarbonate resins, polyurethane resins, polyvinyl chloride resins, polyether ether ketone resins, epoxy resins, phenol resins, and Noryl resins, ceramics such as zirconia, and glass such as borosilicate glass and quartz glass are preferable as the material of the column main body. Moreover, the material of the end fitting may be the same as the material of the column main body or different from the material of the column main body.

The shape of the column main body is a shape used for the publicly known column for liquid chromatography, and a cylindrical shape is preferable.

The preferable lower limit value of the inner diameter of the column main body is 0.5 mm, and the preferable upper limit value is 10 mm. When the inner diameter of the column main body is less than 0.5 mm, it becomes difficult to keep the pressure value within the specified pressure value for the second invention or the fifth invention and the reproducibility may be deteriorated. When the inner diameter of the column main body exceeds 10 mm, the diffusion of the measurement sample or the mobile phase in the column becomes large and the separation performance may be deteriorated. More preferable lower limit value of the inner diameter of the column main body is 1.0 mm, and more preferable upper limit value is 6.0 mm.

The preferable lower limit value of the length of the column main body is 5 mm, and the preferable upper limit value is 100 mm. When the length of the column main body is less than 5 mm, the amount of the packing material interacting with the measurement sample is small, and therefore the separation performance may be deteriorated. When the length of the column main body exceeds 100 mm, the elution of hemoglobin requires time, and therefore the measuring time may be extended. More preferable lower limit value of the length of the column main body is 10 mm, and more preferable upper limit value is 50 mm.

The silicone-treated column used in the second invention and the fifth invention has the above-described column main body whose inner surface is treated with the silicone solution. It is preferable that the silicone solution used in the second invention and the fifth invention is the silicone solution similar to the silicone solution in the first invention and the fourth invention.

The treatment of the column main body with the silicone solution is similar to the treatment of the filter according to the first invention and the fourth invention. Namely, the treatment having a step of bringing the column main body into contact with the silicone solution and a step of covering the inner surface of the column main body by the silicone in the silicone solution brought into contact with the column main body is preferable.

Examples of the method for bringing the column main body into contact with the silicone solution include a method of immersing the column main body into the silicone solution, a method of passing the silicone solution through the column main body, and a method of coating the column main body with the silicone solution.

As a method for curing the silicone in the silicone solution brought into contact with the column main body, a method similar to the method according to the first invention and the fourth invention can be used.

Moreover, the similar treatment with the silicone solution may also be applied to the end fitting of the column in addition to the column main body.

Moreover, blocking treatment may be applied to the silicone-treated column used in the second invention and the fifth invention with a blocking reagent. A blocking reagent to be used and a method for blocking treatment are similar to the blocking reagent and the method for blocking treatment according to the first invention and the fourth invention.

The second invention is a method for measuring stable hemoglobin A1c using liquid chromatography, comprising installing the silicone-treated column on a flow path of a liquid chromatograph. Moreover, the fifth invention is a method for simultaneous measurement of stable hemoglobin A1c and abnormal hemoglobin using liquid chromatography, comprising installing the silicone-treated column on a flow path of a liquid chromatograph.

The liquid chromatography is carried out connecting the silicone-treated column packed with a packing material to the flow path (reference numeral 7 in Fig. 1) of the liquid chromatograph in Fig. 1.

It is preferable that the packing material for packing the silicone-treated column is a packing material having an ion exchange group, more preferably a packing material having a cation exchange group.

A carboxyl group, a phosphate group, a sulfonate group, and so on are preferable as the cation exchange group, and above all, the sulfonate group is preferable.

It is preferable that the lower limit of the average particle diameter value of the packing material particle is 1 µm and the upper limit is 50 µm. In the case where the average particle diameter value of the packing material particle is less than 1 µm, it may become difficult to keep the pressure value within the specified pressure value and the reproducibility may be deteriorated. When the average particle diameter value of the packing material particle exceeds 50 µm, the measurement sample is diffused between the packing material particles and the separation performance may be deteriorated. More preferable lower limit value of the average particle diameter value of the packing material particle is 2 µm, and more preferable upper limit value is 30 µm.

In addition, the average particle diameter value of the packing material particle can be measured using a particle size distribution measuring apparatus.

In the second invention and the fifth invention, the pressure value generated in the measurement system is set to 9.8 × 10³ Pa or more and 19.6 × 10⁵ Pa or less. In the case where the pressure value is set to less than 9.8 × 10³ Pa, the setting pressure value is very small, and therefore it becomes difficult to stabilize the pressure value. Moreover, it may occur that the measuring time becomes long. In the case where the pressure value is set to a value exceeding 19.6 × 10⁵ Pa, the reproducibility is deteriorated.

The liquid chromatograph used in the second invention and the fifth invention is similar to the liquid chromatograph used in the first invention and the fourth invention. Moreover, the eluent used in the second invention and the fifth invention is similar to the eluent used in the first invention and the fourth invention.

Stable hemoglobin A1c and other hemoglobins contained in the healthy human blood can be measured by the second invention in the same manner as in the first invention. Moreover, stable hemoglobin A1c, other hemoglobins contained in the healthy human blood, and hemoglobins generally called as abnormal hemoglobin can be simultaneously measured by the fifth invention in the same manner as in the fourth invention.

The third invention of the present invention is a method for measuring stable hemoglobin A1c using liquid chromatography, comprising installing on a flow path of a liquid chromatograph a filter whose surface is treated with a solution containing 1 to 50% by weight of a silicone oil or a solution containing 1 to 50% by weight of a silicone resin, and a column main body whose inner surface is treated with a solution containing 1 to 50% by weight of a silicone oil or a solution containing 1 to 50% by weight of a silicone resin; and setting a pressure value generated in a measurement system of the liquid chromatograph to 9.8 × 10³ Pa or more and 19.6 × 10⁵ Pa or less.

In the third invention of the present invention, since both of the silicone-treated filter in the first invention and the silicone-treated column in the second invention are installed on the flow path of the liquid chromatograph and the pressure value generated in the measurement system is set to the specified pressure value of the present invention, stable hemoglobin A1c can be measured in a further shorter time with good reproducibility.

Moreover, the sixth invention of the present invention is a method for simultaneous measurement of stable hemoglobin A1c and abnormal hemoglobin using liquid chromatography, comprising installing on a flow path of a liquid chromatograph a filter whose surface is treated with a solution containing 1 to 50% by weight of a silicone oil or a solution containing 1 to 50% by weight of a silicone resin, and a column main body whose inner surface is treated with a solution containing 1 to 50% by weight of a silicone oil or a solution containing 1 to 50% by weight of a silicone resin; and setting a pressure value generated in a measurement system of the liquid chromatograph to 9.8 × 10³ Pa or more and 19.6 × 10⁵ Pa or less.

In the sixth invention of the present invention, since both of the silicone-treated filter in the fourth invention and the silicone-treated column in the fifth invention are used and the pressure value generated in the measurement system is set to the specified pressure value of the present invention, stable hemoglobin A1c and abnormal hemoglobin can be measured in a further shorter time with good reproducibility.

### - Advantageous Effects of Invention

According to the present invention, the measurement of stable hemoglobin A1c and the simultaneous measurement of stable hemoglobin A1c and abnormal hemoglobin can be carried out in a short time with good reproducibility by, in liquid chromatography, using at least one of a filter treated with a silicone solution containing a specific concentration of silicone and a column main body treated with a silicone solution; and setting the pressure value to 9.8 × 10³ Pa or more and 19.6 × 10⁵ Pa or less.

### BRIEF DESCRIPTION OF DRAWINGS

Fig. 1 is a configuration diagram illustrating an example of a liquid chromatograph used in the present invention.
Fig. 2 is a chromatogram obtained by Measurement Example 1 in Evaluation (3).
Fig. 3 is a chromatogram obtained by Measurement Example 8 in Evaluation (3).
Fig. 4 is a chromatogram obtained by Measurement Example 1 in Evaluation (5).
Fig. 5 is a chromatogram obtained by Measurement Example 8 in Evaluation (5).
Fig. 6 is a chromatogram obtained by Measurement Example 1 in Evaluation (6).
Fig. 7 is a chromatogram obtained by Measurement Example 8 in Evaluation (6).
Fig. 8 is a graph showing the relation between the average pressure value and the CV value of the pressure value of Measurement Examples 13, 14, and 15 in Evaluation (7).
Fig. 9 is a graph showing the relation between the average pressure value and the CV value of the pressure value of Measurement Examples 16, 17, and 18 in Evaluation (7).
Fig. 10 is a graph showing the relation between the average pressure value and the CV value of the stable hemoglobin A1c (stable HbA1c) value of Measurement Examples 13, 14, and 15 in Evaluation (8).
Fig. 11 is a graph showing the relation between the average pressure value and the CV value of the stable hemoglobin A1c (stable HbA1c) value of Measurement Examples 16, 17, and 18 in Evaluation (8).
Fig. 12 is a graph showing the relation between the average pressure value and the CV value of the pressure value of Measurement Examples 13, 14, and 15 in Evaluation (9).
Fig. 13 is a graph showing the relation between the average pressure value and the CV value of the pressure value of Measurement Examples 16, 17, and 18 in Evaluation (9).
Fig. 14 is a graph showing the relation between the average pressure value and the CV value of the stable hemoglobin A1c (stable HbA1c) value of Measurement Examples 13, 14, and 15 in Evaluation (10).
Fig. 15 is a graph showing the relation between the average pressure value and the CV value of the stable hemoglobin A1c (stable HbA1c) value of Measurement Examples 16, 17, and 18 in Evaluation (10).
Fig. 16 is a graph showing the relation between the average pressure value and the CV value of the hemoglobin S value of Measurement Examples 13, 14, and 15 in Evaluation (10).
Fig. 17 is a graph showing the relation between the average pressure value and the CV value of the hemoglobin S value of Measurement Examples 16, 17, and 18 in Evaluation (10).
Fig. 18 is a graph showing the relation between the average pressure value and the CV value of the hemoglobin C value of Measurement Examples 13, 14, and 15 in Evaluation (10).
Fig. 19 is a graph showing the relation between the average pressure value and the CV value of the hemoglobin C value of Measurement Examples 16, 17, and 18 in Evaluation (10).
Fig. 20 is a graph showing the shift of the average pressure value at the time of repeated measurement of Measurement Examples 16, 19, and 20 in Evaluation (11).
Fig. 21 is a graph showing the shift of the CV value of the pressure value at the time of repeated measurement of Measurement Examples 16, 19, and 20 in Evaluation (11).
Fig. 22 is a graph showing the shift of the CV value of the stable hemoglobin A1c (stable HbA1c) value at the time of repeated measurement of Measurement Examples 16, 19, and 20 in Evaluation (11).
Fig. 23 is a graph showing the shift of the average pressure value at the time of repeated measurement of Measurement Examples 16, 19, and 20 in Evaluation (12).
Fig. 24 is a graph showing the shift of the CV value of the pressure value at the time of repeated measurement of Measurement Examples 16, 19, and 20 in Evaluation (12).
Fig. 25 is a graph showing the shift of the CV value of the stable hemoglobin A1c (stable HbA1c) value at the time of repeated measurement of Measurement Examples 16, 19, and 20 in Evaluation (12).
Fig. 26 is a graph showing the shift of the CV value of the hemoglobin S value at the time of repeated measurement of Measurement Examples 16, 19, and 20 in Evaluation (12).
Fig. 27 is a graph showing the shift of the CV value of the hemoglobin C value at the time of repeated measurement of Measurement Examples 16, 19, and 20 in Evaluation (12).

### DESCRIPTION OF EMBODIMENTS

Hereinafter, the present invention will be described in more detail giving Examples, however the present invention is not limited to only these Examples.

### (Production Example 1)

In Production Examples 1 to 3, column packing materials for measuring hemoglobins, the column packing materials each having different average particle diameter value were prepared.

In a monomer mixture of 150 g of tetraethylene glycol dimethacrylate (crosslinkable monomer, manufactured by Shin-Nakamura Chemical Co., Ltd.), 140 g of triethylene glycol dimethacrylate (crosslinkable monomer, manufactured by Shin-Nakamura Chemical Co., Ltd.), and 60 g of 2-hydroxy-1,3-dimethacryloxy propane (crosslinkable monomer, manufactured by Shin-Nakamura Chemical Co., Ltd.), 1.0 g of benzoyl peroxide (manufactured by Kishida Chemical Co., Ltd.) as a polymerization initiator was mixed and dissolved, and the resultant mixture was dispersed in 2000 mL of a 5% by weight aqueous solution of polyvinyl alcohol ("GOHSENOL GH-20" manufactured by The Nippon Synthetic Chemical Industry Co., Ltd.). Polymerization reaction was carried out for 1.2 hours raising the temperature to 80°C under a nitrogen atmosphere while the reaction system was stirred at 350 rpm. Next, 200 mL of ion exchanged water in which 180 g of 2-acrylamide-2-methylpropane sulfonic acid (hydrophilic monomer, manufactured by Toagosei Co., Ltd.), 60 g of polyethylene glycol monomethacrylate (hydrophilic monomer, manufactured by NOF Corporation), and 100 g of methanol were dissolved was added to the reaction system, and polymerization reaction was carried out at 80°C for 2 hours. The obtained polymer was washed and a column packing material was obtained.

The average particle diameter was measured by a particle size distribution measuring apparatus ("Accusizer 780" manufactured by Nicomp International Inc.), and as a result thereof, the average particle diameter was 4.7 µm.

### (Production Example 2)

A column packing material was obtained by carrying out the same operation as in Production Example 1 except that the stirring condition of the reaction system in Production Example 1 was changed to 300 rpm. As a result of measuring the average particle diameter in the same manner as in Production Example 1, the average particle diameter was 6.4 µm.

### (Production Example 3)

A column packing material was obtained by carrying out the same operation as in Production Example 1 except that the stirring condition of the reaction system in Production Example 1 was changed to 250 rpm. As a result of measuring the average particle diameter in the same manner as in Production Example 1, the average particle diameter was 9.5 µm.

The average particle diameter values of the column packing materials obtained in the above-described Production Examples 1 to 3 are shown in Table 1.

**[Table 1]**

| | Average particle diameter (*µ*m) |
|---|---|
| Production Example 1 | 4.7 |
| Production Example 2 | 6.4 |
| Production Example 3 | 9.5 |

### (Example 1)

In Examples 1 to 3, silicone-treated filters were prepared.

A columnar filter having a diameter of 4 mm and a thickness of 1.5 mm was produced as an integrally molded article of a sintered stainless steel body (hereinafter, this filter is also referred to as the "untreated filter"). An ethyl alcohol solution containing 3% by weight of silicone ("KF-96" manufactured by Shin-Etsu Chemical Co., Ltd.) was used as a silicone solution. The filter was immersed in the silicone solution under room temperature for 30 minutes, thereafter the filter was taken out from the silicone solution. Then, the silicone was cured by heating the filter at 100°C for 60 minutes and a silicone-treated filter was obtained.

### (Example 2)

Silicone treatment was performed applying as the silicone solution an ethyl alcohol solution containing 20% by weight of silicone ("SR 2410" manufactured by Dow Corning Toray Co., Ltd.) in place of the ethyl alcohol solution containing 3% by weight of silicone in Example 1.

The untreated filter used in Example 1 was immersed in the silicone solution under room temperature for 30 minutes, thereafter the filter was taken out from the silicone solution. Then, the silicone was cured by heating the filter at 120°C for 100 minutes and a silicone-treated filter was obtained.

### (Example 3)

Silicone treatment was performed applying as the silicone solution a butyl alcohol solution containing 45% by weight of silicone ("SR 2309" manufactured by Dow Corning Toray Co., Ltd.) in place of the ethyl alcohol solution containing 3% by weight of silicone in Example 1.

The untreated filter used in Example 1 was immersed in the silicone solution under room temperature for 30 minutes, thereafter the filter was taken out from the silicone solution. Then, the silicone was cured by heating the filter at 120°C for 100 minutes and a silicone-treated filter was obtained.

### (Comparative Example 1)

In Comparative Example 1, a filter not treated with silicone was prepared.

As a filter not treated with silicone, the untreated filter used in Example 1 was used as it was without being treated with a silicone solution.

### (Comparative Example 2)

In Comparative Example 2, a filter treated with a silicone solution containing a silicone at a concentration less than the concentration specified in the first invention and the fourth invention of the present invention was prepared.

Silicone treatment was performed applying as the silicone solution an ethyl alcohol solution containing 0.5% by weight of silicone ("KF-96" manufactured by Shin-Etsu Chemical Co., Ltd.) in place of the ethyl alcohol solution containing 3% by weight of silicone in Example 1.

The untreated filter used in Example 1 was immersed in the silicone solution under room temperature for 30 minutes, thereafter the filter was taken out from the silicone solution. Then, the silicone was cured by heating the filter at 120°C for 100 minutes and a silicone-treated filter was obtained.

### (Comparative Example 3)

In Comparative Example 3, a filter treated with a silicone solution containing silicone at a concentration exceeding the concentration specified in the first invention and the fourth invention of the present invention was prepared.

Silicone treatment was performed applying as the silicone solution an ethyl alcohol solution containing 70% by weight of silicone ("SR 2410" manufactured by Dow Corning Toray Co., Ltd.) in place of the ethyl alcohol solution containing 20% by weight of silicone in Example 2.

The untreated filter used in Example 1 was immersed in the silicone solution under room temperature for 30 minutes, thereafter the filter was taken out from the silicone solution. Then, the silicone was cured by heating the filter at 120°C for 100 minutes and a silicone-treated filter was obtained.

### (Example 4)

In Examples 4 and 5, silicone-treated columns were prepared.

A column main body made of stainless steel SUS 314 (manufactured by Tomoe Works Co., Ltd., length 35 mm and inner diameter 4.6 mm, hereinafter also referred to as an "untreated column main body"), was immersed in an ethyl alcohol solution containing 3% by weight of silicone ("KF-96" manufactured by Shin-Etsu Chemical Co., Ltd.) as a silicone solution under room temperature for 30 minutes. Thereafter, the column main body was taken out from the silicone solution. Then, the silicone was cured by heating the column main body at 100°C for 60 minutes and a silicone-treated column main body was obtained. An end fitting (silicone-untreated) made of stainless steel SUS 314 to which the untreated filter used in Example 1 was fitted as a frit was attached to both ends of the silicone-treated column main body and a silicone-treated column was obtained.

### (Example 5)

The untreated column main body and the end fitting used in Example 4 were immersed in a butyl alcohol solution containing 45% by weight of silicone ("SR 2309" manufactured by Dow Corning Toray Co., Ltd.) as a silicone solution under room temperature for 30 minutes. Thereafter, the column main body and the end fitting were taken out from the silicone solution. Then, the silicone was cured by heating the column main body and the end fitting at 120°C for 100 minutes and a silicone-treated column was obtained.

### (Comparative Example 4)

In Comparative Example 4, a column not treated with a silicone solution was prepared.

As a column not treated with a silicone solution, the untreated column and the end fitting used in Example 4 were used as they were without being treated with a silicone solution.

### (Comparative Example 5)

In Comparative Example 5, a column treated with a silicone solution containing silicone at a concentration less than the concentration specified in the second invention and the fifth invention of the present invention was prepared.

Silicone treatment was performed applying as the silicone solution an ethyl alcohol solution containing 0.5% by weight of silicone ("KF-96" manufactured by Shin-Etsu Chemical Co., Ltd.) in place of the ethyl alcohol solution containing 3% by weight of silicone in Example 4.

The untreated column main body used in Example 4 was immersed in the silicone solution under room temperature for 30 minutes, thereafter the column main body was taken out from the silicone solution. Then, the silicone was cured by heating the column main body at 120°C for 100 minutes and a silicone-treated column main body was obtained. An end fitting (silicone-untreated) made of stainless steel SUS 314 to which the untreated filter used in Example 1 was fitted as a frit was attached to both ends of the silicone-treated column main body and a silicone-treated column was obtained.

### (Comparative Example 6)

In Comparative Example 6, a column treated with a silicone solution containing silicone at a concentration exceeding the concentration specified in the second invention and the fifth invention of the present invention was prepared.

Silicone treatment was performed applying as the silicone solution an ethyl alcohol solution containing 70% by weight of silicone ("KF-96" manufactured by Shin-Etsu Chemical Co., Ltd.) in place of the ethyl alcohol solution containing 3% by weight of silicone in Example 4.

The untreated column main body used in Example 4 was immersed in the silicone solution under room temperature for 30 minutes, thereafter the column main body was taken out from the silicone solution. Then, the silicone was cured by heating the column main body at 120°C for 100 minutes and a silicone-treated column main body was obtained. An end fitting (silicone-untreated) made of stainless steel SUS 314 to which the untreated filter used in Example 1 was fitted was attached to both ends of the silicone-treated column main body and a silicone-treated column was obtained.

The contents of treatment in Examples 1 to 5 and Comparative Examples 1 to 6 are shown in Table 2.

**[Table 2]**

| | Contents of treatment | Silicone concentration (% by weight) |
|---|---|---|
| Example 1 | Silicone treatment on filter | 3 |
| Example 2 | Silicone treatment on filter | 20 |
| Example 3 | Silicone treatment on filter | 45 |
| Comparative Example 1 | No silicone treatment (untreated filter) | - |
| Comparative Example 2 | Silicone treatment on filter | 0.5 |
| Comparative Example 3 | Silicone treatment on filter | 70 |
| Example 4 | Silicone treatment on column main body | 3 |
| Example 5 | Silicone treatment on column main body and frit-containing end fitting | 45 |
| Comparative Example 4 | No silicone treatment (untreated column main body) | - |
| Comparative Example 5 | Silicone treatment on column main body | 0.5 |
| Comparative Example 6 | Silicone treatment on column main body | 70 |

### (Evaluation)

The filters and the columns obtained in Examples 1 to 5 and Comparative Examples 1 to 6 were combined to prepare the measurement systems in Measurement Examples 1 to 12 shown in Table 3.

In addition, the "pre-filter," the "frit," and the "column main body" in Table 3 to which: the treatment was applied with the silicone solution having a concentration within the specified range of the present invention were represented by "○"; the treatment was applied with the silicone solution having a concentration out of the specified range of the present invention were represented by "Δ"; and the treatment was not applied were represented by "×".

**[Table 3]**

| | Silicone-treated components | | | | | | Column packing material | Evaluation results | | | | | | | | | | |
|---|---|---|---|---|---|---|---|---|---|---|---|---|---|---|---|---|---|---|
| | Pre-filter | | Frit | | Column main body | | | Evaluation (3) Measurement of stable HbA1c | | Evaluation (4) Measurement of samples containing modified Hb | | | Evaluation (5) Measurement of samples containing abnormal Hb | | | | Evaluation (6) Measurement of sample containing HbA2 | |
| | | | | | | | | Pressure value (×10³Pa) | Chromatogram | Δ value (%) | | | CV value of simultaneous reproducibililty (%) | | | Chromatogram | simultaneous CV value of simultaneous reproducibility (%) | Chromatogram |
| | | | | | | | | | | Sample L | Sample A | Sample C | Stable HbA1c | HbS | HbC | | HbA2 | |
| Measurement Example 1 | ○ | Example 1 | × | Comparative Example 1 | × | Comparative Example 4 | Production Example 1 | 863 | Fig. 2 | 0.11 | 0.13 | 0.12 | 0.61 | 0. 74 | 0.84 | Fig. 4 | 0.75 | Fig. 6 |
| Measurement Example 2 | ○ | Example 2 | × | Comparative Example 1 | × | Comparative Example 4 | Production Example 1 | 975 | Fig. 2 | 0.16 | 0.14 | 0.12 | 0.72 | 0.86 | 0.92 | Fig. 4 | 0.75 | Fig. 6 |
| Measurement Example 3 | ○ | Example 3 | × | Comparative Example 1 | × | Comparative Example 4 | Production Example 1 | 791 | Fig. 2 | 0.14 | 0.15 | 0.15 | 0.87 | 0.91 | 0.93 | Fig. 4 | 0.86 | Fig. 6 |
| Measurement Example 4 | × | Comparative Example 1 | ○ | Example 1 | × | Comparative Example 4 | Production Example 1 | 865 | Fig. 2 | 0.12 | 0.17 | 0.18 | 0.96 | 0.94 | 0.89 | Fig. 4 | 0.87 | Fig. 6 |
| Measurement Example 5 | × | Comparative Example 1 | × | Comparative Example 1 | ○ | Example 4 | Production Example 1 | 877 | Fig. 2 | 0.16 | 0.15 | 0.19 | 0.95 | 0.95 | 0.90 | Fig. 4 | 0.93 | Fig. 6 |
| Measurement Example 6 | × | Comparative Example 1 | ○ | Example 5 | ○ | Example 5 | Production Example 1 | 1491 | Fig. 2 | 0.11 | 0.12 | 0.12 | 0.76 | 0.89 | 0.93 | Fig. 4 | 0.90 | Fig. 6 |
| Measurement Example 7 | ○ | Example 2 | ○ | Example 5 | ○ | Example 5 | Production Example 1 | 1522 | Fig. 2 | 0.08 | 0.05 | 0.07 | 0.63 | 0.75 | 0.73 | Fig. 4 | 0.92 | Fig. 6 |
| Measurement Example 8 | × | Comparative Example 1 | × | Comparative Example 1 | × | Comparative Example 4 | Production Example 1 | 1296 | Fig. 3 | 0.34 | 0.57 | 0.42 | 4.32 | 5.34 | 4.97 | Fig. 5 | 6.43 | Fig. 7 |
| Measurement Example 9 | Δ | Comparative Example 2 | × | Comparative × Example 1 | × | Comparative Example 4 | Production Example 1 | 1022 | Fig. 3 | 0.39 | 0.54 | 0.45 | 3.21 | 3.87 | 4.23 | Fig. 5 | 5.41 | Fig. 7 |
| Mesurement Example 10 | Δ | Comparative Example 3 | × | Comparative Example 1 | × | Comparative Example 4 | Production Example 1 | 4376 | Fig. 3 | 0.45 | 0.51 | 0.63 | 3.49 | 4.85 | 3.75 | Fig. 5 | 5.32 | Fig. 7 |
| Measurement Example 11 | × | Comparative Example 1 | × | Comparative Example 1 | Δ | Comparative Example 5 | Production Example 1 | 1306 | Fig. 3 | 0.41 | 0.49 | 0.53 | 4.23 | 4.76 | 4.34 | Fig. 5 | 4.95 | Fig. 7 |
| Measurement Example 12 | × | Comparative Example 1 | × | Comparative Example 1 | Δ | Comparative Example 6 | Production Example 1 | 3188 | Fig. 3 | 0.35 | 0.53 | 0.42 | 3.98 | 3.96 | 4.56 | Fig. 5 | 4.92 | Fig. 7 |

### (1) Preparation of Column for Measuring Hemoglobins

The column packing materials obtained in Production Examples 1 to 3 were packed in the columns obtained in Examples and Comparative Examples to prepare columns for measuring hemoglobins.

To 30 mL of a 50 mmol/L phosphate buffer solution (pH 6.0), 0.8 g of each of the column packing materials obtained in Production Examples 1 to 3 was added, and the resultant mixture was stirred, thereafter ultrasonic treatment was applied for 5 minutes to prepare slurry of the packing material. The whole amount of the slurry was injected into a packer for column packing (volume 30 mL, manufactured by AS ONE Corporation) to which each of the columns prepared in Example 4 or 5, or Comparative Example 4, 5, or 6 was connected. A liquid feeding pump ("PU-614" manufactured by GL Sciences Inc.) was connected to the packer, and the column was packed at a pressure of 20 MPa and a column for measuring hemoglobins was obtained.

### (2) Measurement Conditions

The column for measuring hemoglobins obtained by the above-described "(1) Preparation of Column for Measuring Hemoglobins" was connected to a liquid chromatograph 1 in Fig. 1 (reference numeral 7 in Fig. 1). Moreover, each filter of Examples 1 to 3, or Comparative Examples 1 to 3 was connected between an injection valve 5 and a separation column 7 (reference numeral 6 in Fig. 1). The pressure value was measured connecting a pressure gauge 4 ("Digital Pressure Gauge GC 61" manufactured by Nagano Keiki Co., Ltd.) between a liquid feeding pump 3 and the injection valve 5.

Measurement conditions are shown in Table 4.

**[Table 4]**

| System | LC-20A system (SHIMADZU CORP.) |
|---|---|
| Eluent | Eluent A: 100 mmol/L phosphate buffer solution (pH 5.8) |
| | Eluent B: 300 mmol/L phosphate buffer solution (pH 6.8) |
| Conditions for elution | 0.0 min to 0.6 min: Eluent A 100% |
| | 0.6 min to 0.7 min: Eluent B 100% |
| | 0.8 min to 1.0 min: Eluent A 100% |
| Flow rate | 1.6 mL/min |
| Detection wavelength | 415 nm |
| Amount of sample injected | 10 *µ*L |

### Evaluation (3) Measurement of Stable Hemoglobin A1c

Stable hemoglobin A1c in the healthy human blood was measured using the measurement systems in Measurement Examples 1 to 12 under the measurement conditions described in Table 4.

A measurement sample obtained by 1:120 hemolytic dilution of healthy human blood collected with a blood-collecting vessel containing sodium fluoride by a phosphate buffer solution (pH 6.7) containing 0.05% Triton X-100 (manufactured by Sigma-Aldrich Japan K.K.) was used.

A chromatogram obtained by Measurement Example 1 is shown in Fig. 2. Moreover, the pressure value at the time of the measurement is shown in Table 3. In the case where the measurement was carried out using the measurement system of Measurement Example 1, stable hemoglobin A1c (peak 1) was able to be favorably separated from the other hemoglobins within 1 minute. Also in the cases where the measurement systems of Measurement Examples 2 to 7 were used, stable hemoglobin A1e was able to be favorably separated, and chromatograms similar to the chromatogram in Fig. 2 were obtained.

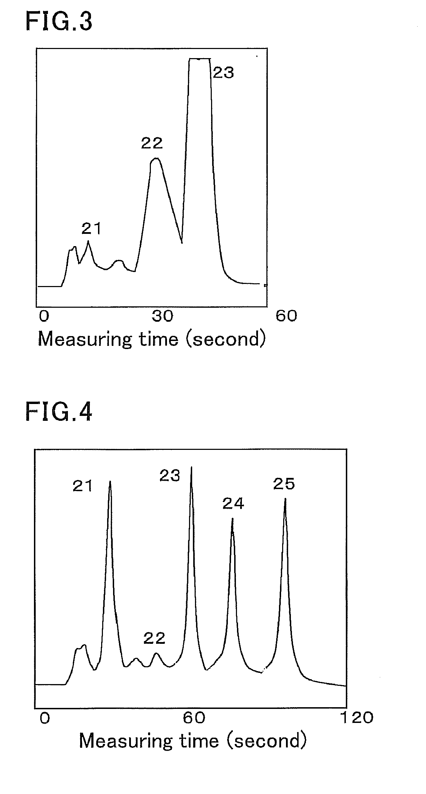

A chromatogram obtained by Measurement Example 8 in which the silicone-treated filter and the silicone-treated column were not used is shown in Fig. 3. In the case where the measurement was carried out using the measurement system of Measurement Example 8, the separation performance was worse compared with the case where the measurement was carried out using the measurement system of Measurement Example 1 and stable hemoglobin A1c was not able to be separated.

Moreover, the cases of Measurement Examples 9 and 11 where the filter or the column treated with the silicone solution having a concentration less than the specified amount in the present invention was used also showed chromatograms similar to the chromatogram in Fig. 3. This is because the silicone concentration was low and therefore the effect of silicone treatment was not exhibited.

The cases of Measurement Examples 10 and 12 where the filter or the column main body was treated with the silicone solution having a silicone concentration exceeding the specified amount in the present invention also showed chromatograms similar to the chromatogram in Fig. 3. It is thought that this is because the pressure value by the treatment with high-concentration silicone solution exceeded the specified value and therefore the separation performance was not improved.

### Evaluation (4) Measurement of Modified Hemoglobins

Samples containing modified hemoglobin artificially prepared were measured using measurement systems of Measurement Examples 1 to 12 under the measurement conditions described in Table 4. As a sample containing modified hemoglobin, three kinds of samples, a sample containing unstable hemoglobin A1c (Sample L), a sample containing acetylated hemoglobin (Sample A), and a sample containing carbamylated hemoglobin (Sample C), were prepared by a publicly known method.

Sample L was prepared by adding glucose to the healthy human blood so as to give a glucose concentration of 2000 mg/dL and by heating the resultant mixture at 37°C for 3 hours. Sample A was prepared by adding acetaldehyde to the healthy human blood so as to give a acetaldehyde concentration of 50 mg/dL and heating the resultant mixture at 37°C for 2 hours. Sample C was prepared by adding sodium cyanate to the healthy human blood so as to give a sodium-cyanate concentration of 50 mg/dL and heating the resultant mixture at 37°C for 2 hours.

The separation performance was evaluated by calculating and comparing a value (Δ value) obtained by subtracting the stable hemoglobin A1c value of the healthy human blood (non-modified product) used for the preparation of the sample containing modified hemoglobin from the stable hemoglobin A1c value of the sample containing modified hemoglobin (Sample L, Sample A, or Sample C). The results are shown in Table 3.

The Δ values in the cases of Measurement Examples 1 to 7 where the silicone-treated filter or the silicone-treated column was used were less than 0.2%, and stable hemoglobin A1c was able to be measured accurately even for the sample containing modified hemoglobin. Particularly, the Δ value of Measurement Example 7 in which both of the silicone-treated filter and the silicone-treated column were used was small, and it was understood that Measurement Example 7 was excellent in the separation performance for stable hemoglobin A1c.

The Δ value in the case of Measurement Example 8 where the silicone-untreated filter and the silicone-untreated column were used was 0.3% or more, and accurate measurement of stable hemoglobin A1c was not able to be carried out affected by the modified hemoglobin.

The Δ values in the cases of Measurement Examples 9 and 11 where the filter or the column treated with the silicone solution having a concentration less than the specified amount of the present invention was used and the Δ values in the cases of Measurement Examples 10 and 12 where the filter or the column main body were treated with the silicone solution having a silicone concentration exceeding the specified amount of the present invention were also 0.3% or more, and accurate measurement of stable hemoglobin A1c was not able to be carried out affected by the modified hemoglobins.

### Evaluation (5) Simultaneous Measurement of Stable Hemoglobin A1c and Abnormal Hemoglobin

A sample ("AFSC hemocontrol" manufactured by Helena Laboratories) containing hemoglobin S and hemoglobin C as abnormal hemoglobin was measured using the measurement systems of Measurement Examples 1 to 12 under the measurement conditions described in Table 5.

In the case of Measurement Example 1 where the silicone-treated filter was used (Fig. 4), hemoglobin S (peak 24) and hemoglobin C (peak 25) were able to be favorably separated.

Also in the cases where the measurement systems of Measurement Examples 2 to 7 were used, chromatograms similar to the chromatogram in Fig. 4 were obtained.

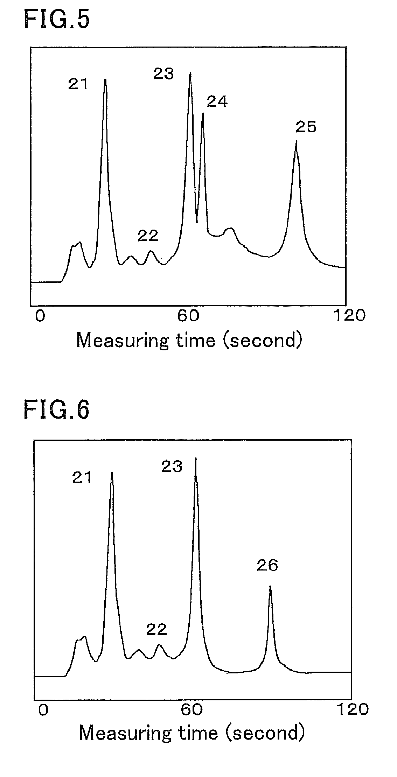

In the case where the measurement system of Measurement Example 8 was used (Fig. 5), hemoglobin S and hemoglobin C were not able to be separated.

Also in the cases where the measurement systems of Measurement Examples 9 to 12 were used, favorable separation was not obtained, as well as the case where the measurement system of Measurement Example 8 was used, and chromatograms similar to the chromatogram in Fig. 5 were obtained.

The measurement of the above sample was repeated 20 times to confirm simultaneous reproducibility of stable hemoglobin A1c, hemoglobin S, and hemoglobin C (Table 3).

Favorable reproducibility was exhibited as the CV values of each hemoglobin of Measurement Examples 1 to 7 were 1% or less.

Reproducibility was unfavorable as the CV values of Measurement Examples 8 to 12 were large.

**[Table 5]**

| System | LC-20A system (SHIMADZU CORP.) |
|---|---|
| Eluent | Eluent A: 100 mmol/L phosphate buffer solution (pH 5.8) |
| | Eluent B: 200 mmol/L phosphate buffer solution (pH 6.8) |
| | Eluent C: 180 mmol/L phosphate buffer solution (pH 6.3) |
| Conditions for elution | 0.0 min to 1.0 min: Eluent A 100% |
| | 1.0 minto 1.1 min: Eluent B 100% |
| | 1.1 min to 1.6 min: Eluent C 100% |
| | 1.6 min to 2.0 min: Eluent A 100% |
| Flow rate | 1.5 mL/min |
| Detection wavelength | 415 nm |
| Amount of sample injected | 10 *µ*L |

### Evaluation (6) Measurement of Hemoglobin A2

A sample ("A2 Control Level 2" manufactured by Bio-Rad Laboratories, Inc.) containing hemoglobin A2 (HbA2) was measured using the measurement systems of Measurement Examples 1 to 7 under the measurement conditions described in Table 5.

In the case of Measurement Example 1 where the silicone-treated filter was used (Fig. 6), hemoglobin A2 (peak 26) was able to be favorably separated.

Also in the cases of Measurement Examples 2 to 7, chromatograms similar to the chromatogram in Fig. 6 were obtained.

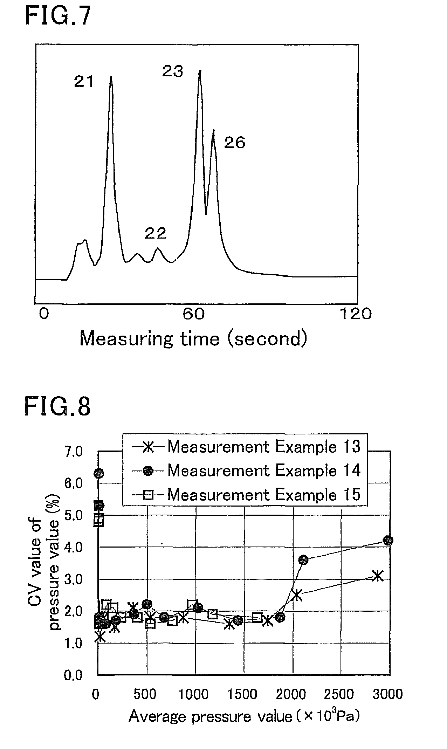

In the case where the measurement system of Measurement Example 8 was used (Fig. 7), hemoglobin A2 was not able to be separated.

Also in the cases where the measurement systems of Measurement Examples 9 to 12 were used, favorable separation was not obtained, as well as the case where the measurement system of Measurement Example 8 was used, and chromatograms similar to the chromatogram in Fig. 7 were obtained.

The measurement of the above sample was repeated 20 times to confirm simultaneous reproducibility of hemoglobin A2 (Table 3).

Favorable reproducibility was exhibited as the CV values of Measurement Examples 1 to 7 were 1% or less.

Reproducibility was unfavorable as the CV values of Measurement Examples 8 to 12 were large.

By the evaluation (3) to (6), it was confirmed that the separation performance for hemoglobins was improved by using at least any one of the filter and the column main body treated with the silicone solution containing the specified concentration of the present invention of silicone.

### Evaluation (7) Evaluation of Pressure Value 1

In evaluation (7) to (10), measurement was carried out changing the pressure value of the measurement systems by intentionally changing the flow rate at the time of the measurement. Conditions of Measurement Examples 13 to 20 used for the evaluation of the pressure value are shown in Table 6 and Table 7.

In addition, the "pre-filter," the "frit," and the "column main body" in Table 6 to which: the treatment was applied with the silicone solution having a concentration within the specified range of the present invention were represented as "○;" and the treatment with silicone was not applied were represented as "×."

**[Table 6]**

| | Silicone-treated components | | | | | | Column packing material | Evaluation results | | | | |
|---|---|---|---|---|---|---|---|---|---|---|---|---|
| | | | | | | | | Evaluation (7) Pressure value | Evaluation (8) Simultaneous reproducibility | Evaluation (11) Repeated measurement | | |
| | Pre-filter | | Frit | | Column main body | | | CV value of pressure value | CV value of stable HbA1c value | Average pressure value | CV value of pressure value | CV value of stable HbA1c vatue |
| Measurement Example 13 | ○ | Example 2 | × | Comparative Example 1 | × | Comparative Example 4 | Production Example 1 | Fig. 8 | Fig. 10 | - | - | - |
| Measurement Example 14 | ○ | Example 2 | × | Comparative Example 1 | × | Comparative Example 4 | Production Example 2 | Fig. 8 | Fig. 10 | - | - | - |
| Measurement Example 15 | ○ | Example 2 | × | Comparative Example 1 | × | Comparative Example 4 | Production Example 3 | Fig. 8 | Fig. 10 | - | - | - |
| Measurement Example 16 | × | Comparative Example 1 | × | Comparative Example 1 | × | Comparative Example 4 | Production Example 1 | Fig. 9 | Fig. 11 | Fig. 20 | Fig. 21 | Fig. 22 |
| Measurement Example 17 | × | Comparative Example 1 | × | Comparative Example 1 | × | Comparative Example 4 | Production Example 2 | Fig. 9 | Fig. 11 | - | - | - |
| Measurement Example 18 | × | Comparative Example 1 | × | Comparative Example 1 | × | Comparative Example 4 | Production Example 3 | Fig. 9 | Fig. 11 | - | - | - |
| Measurement Example 19 | ○ | Example 2 | × | Comparative Example 1 | × | Comparative Example 4 | Production Example 2 | - | - | Fig. 20 | Fig. 21 | Fig. 22 |
| Measurement Example 20 | ○ | Example 2 | ○ | Example 4 | ○ | Examples 4 | Production Example 2 | - | - | Fig. 20 | Fig. 21 | Fig. 22 |

**[Table 7]**

| | Silicone-treated components | | | | | | Column packing material | Evaluation results | | | | | | | | |
|---|---|---|---|---|---|---|---|---|---|---|---|---|---|---|---|---|
| | | | | | | | | Evaluation (9) Pressure value | Evaluation (10) Simultaneous reproducibility | | | Evaluation (12) Repeated measurement | | | | |
| | Pre-filter | | Frit | | Column main body | | | CV value of pressure value | CV value of stable HbA1c value | CV value of HbUbS value | CV value of HbC value | Average pressure value | CV value of pressure value | CV value of stable HbA1c value | CV value of HbS value | CV value of HbC value |
| Measurement Example 13 | ○ | Example 2 | × | Comparative Example 1 | × | Comparative Example 4 | Production Example 1 | Fig. 12 | Fig. 14 | Fig. 16 | Fig_{.} 18 | - | - | - | - | - |
| Measurement Example 14 | ○ | Example 2 | × | Comparative Example 1 | × | Comparative Example 4 | Production Example 2 | Fig. 12 | Fig. 14 | Fig. 16 | Fig. 18 | - | - | - | - | - |
| Measurement Example 15 | ○ . | Examples 2 | × | Comparative Example 1 | × | Comparative Example 4 | Production Example 3 | Fig. 12 | Fig. 14 | Fig. 16 | Fig. 18 | - | - | - | - | - |
| Measurement Example 16 | × | Comparative Example 1 | × | Comparative Example 1 | × | Comparative Example 4 | Production Example 1 | Fig. 13 | Fig. 15 | Fig. 17 | Fig 19 | Fig. 23 | Fig. 24 | Fig. 25 | Fig. 26 | Fig. 27 |
| Measurement Example 17 | × | Comparative Example 1 | × | Comparative Example 1 | × | Comparative Example 4 | Production Example 2 | Fig. 13 | Fig. 15 | Fig. 17 | Fig. 19 | - | - | - | - | - |
| Measurement Example 18 | × | Comparative Example 1 | × | Comparative Example 1 | × | Comparative Example 4 | Production Example 3 | Fig. 13 | Fig. 15 | Fig. 17 | Fig. 19 | - | - | - | - | - |
| Measurement Example 19 | ○ | Example 2 Example | × | Comparative Example 1 | × | Comparative Example 4 | Production Example 2 | - | - | - | - | Fig. 23 | Fig. 24 | Fig. 25 | Fig. 26 | Fig. 27 |
| Measurement Example 20 | ○ | Example 2 | ○ | Example 4 | ○ | Example 4 | Production Example 2 | - | - | - | - | Fig. 23 | Fig. 24 | Fig. 25 | Fig. 26 | Fig. 27 |

First of all, stable hemoglobin A1c in the healthy human blood was measured using the packing materials of Production Examples 1 to 3 and the silicone-treated filter of Example 2 by Measurement Examples 13 to 15 under the measurement conditions described in Table 4 except the flow rate.

The relation between the average pressure value and the CV value of the pressure value in the case where measurement was carried out 20 times at each flow rate changing the flow rate from 0.1 to 3.3 mL/min is shown in Fig. 8. The CV value of the pressure value within the specified pressure value of the present invention was favorable and stable.

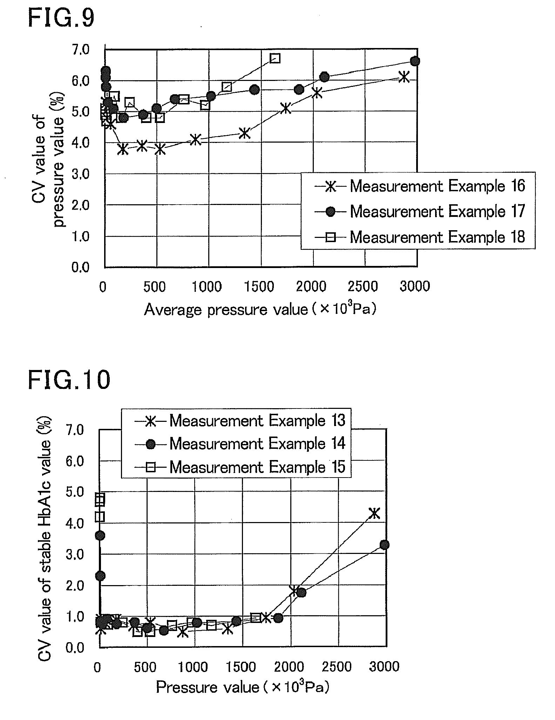

The relation between the average pressure value and the CV value of the pressure value in the cases of Measurement Examples 16 to 18 where the measurement was carried out using the packing materials of Production Examples 1 to 3 and the filter of Comparative Example 1 (silicone-untreated filter) and changing the flow rate in the same way as in Measurement Examples 13 to 15 is shown in Fig. 9. The CV value of the pressure value was large when compared with Fig. 8, and the CV value of the pressure value within the specified pressure value of the present invention was not improved.

It was confirmed from the above results that the favorable CV value of the pressure value within the specified pressure value of the present invention was stably obtained when the silicone-treated filter according to the present invention was used.

### Evaluation (8) Reproducibility Test of Stable Hemoglobin A1c Value

Simultaneous reproducibility of the stable hemoglobin A1c value was confirmed at the time of the "(7) Evaluation of Pressure Value 1." The CV value of the stable hemoglobin A1c value was calculated in the case where a sample of the healthy human blood was measured continuously 20 times at the time of the measurement at each flow rate. The relation between the average pressure value and the CV value of the stable hemoglobin A1c value at the time of measurement of Measurement Examples 13 to 15 of the cases where the silicone-treated filter was used is shown in Fig. 10. Favorable reproducibility was obtained as the CV values were 1.0% or less within the specified pressure value of the present invention. Even in the case where the silicone-treated filter was used, the CV value became large when the pressure value was out of the range of the specified pressure value of the present invention.

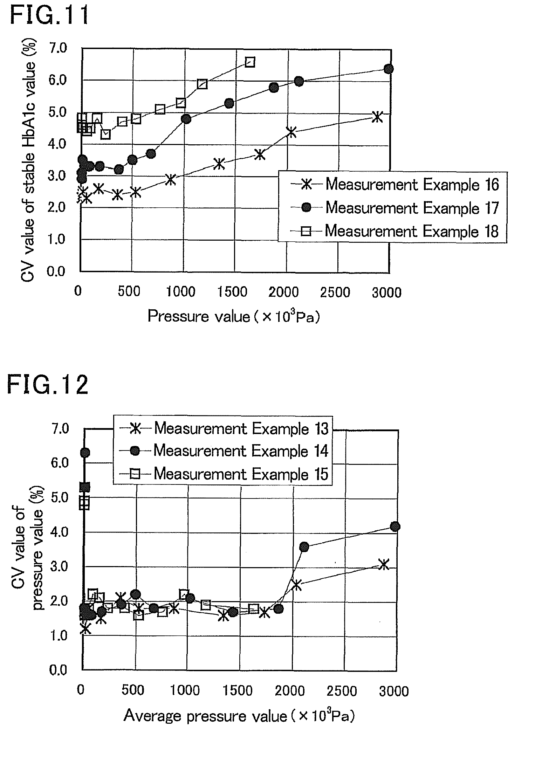

The relation between the average pressure value and the CV value of the stable hemoglobin A1c value at the time of measurement in the cases of Measurement Examples 16 to 18 where the silicone-untreated filter was used is shown in Fig. 11. Reproducibility was unfavorable as the CV values were not 1.0% or less.

It was confirmed from the above results that the pressure value was stable and the reproducibility of measurement of the stable hemoglobin A1c value was improved in the case where the silicone-treated filter was used and the pressure value was made within the specified pressure value of the present invention. In addition, the similar behavior difference was shown when the silicone-treated column and the column not treated with silicone were compared.

### Evaluation (9) Evaluation of Pressure Value 2

First of all, the sample containing abnormal hemoglobin (AFSC hemocontrol) was measured using the packing materials of Production Examples 1 to 3 and the silicone-treated filter of Example 2 by Measurement Examples 13 to 15 under the measurement conditions described in Table 5 except the flow rate.

The relation between the average pressure value and the CV value of the pressure value in the case where measurement was carried out 20 times at each flow rate changing the flow rate from 0.1 to 3.3 mL/min is shown in Fig. 12.

The CV value of the pressure value within the specified pressure value of the present invention was favorable and stable.

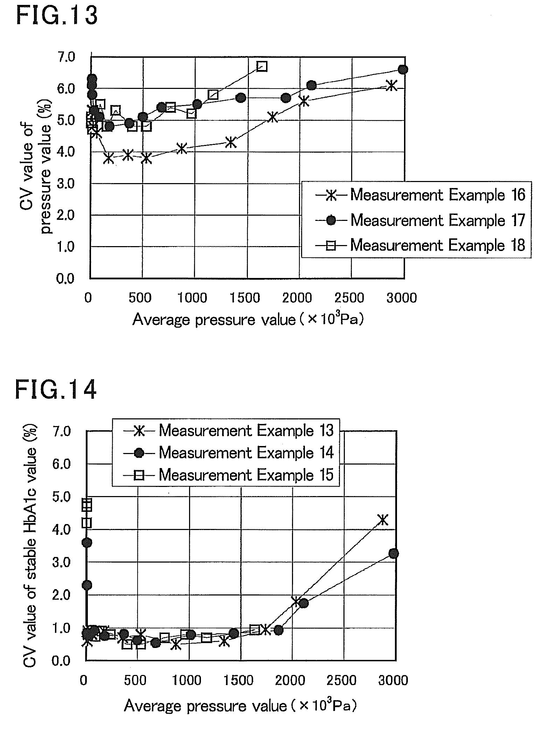

The relation between the average pressure value and the CV value of the pressure value in the cases of Measurement Examples 16 to 18 where the measurement was carried out using the packing materials of Production Examples 1 to 3 and the filter of Comparative Example 1 (silicone-untreated filter) and changing the flow rate in the same way as in Measurement Examples 13 to 15 is shown in Fig. 13.

The CV value of the pressure value was large when compared with Fig. 12, and the CV value of the pressure value within the specified pressure value of the present invention was not improved.

It was confirmed from the above results that the favorable CV value of the pressure value within the specified pressure value of the present invention was stably obtained when the silicone-treated filter according to the present invention was used.

Moreover, it was confirmed that the CV value became worse when the pressure value was out of the range of the specified pressure value of the present invention even in the case where the silicone-treated filter was used.

### Evaluation (10) Reproducibility Test of Stable Hemoglobin A1c Value and Abnormal Hemoglobin Value

Simultaneous reproducibility of the stable hemoglobin A1c value and the abnormal hemoglobin value was confirmed at the time of the "(9) Evaluation of Pressure Value 2."

The CV values of the stable hemoglobin A1c value, the hemoglobin S value, and the hemoglobin C value were calculated in the case where the sample containing abnormal hemoglobin (AFSC hemocontrol) was measured continuously 20 times at the time of the measurement at each flow rate.

The relation between the average pressure value and the CV value of the stable hemoglobin A1c value at the time of measurement in the cases of Measurement Examples 13 to 15 where the silicone-treated filter was used is shown in Fig. 14.

Favorable reproducibility was obtained as the CV values were 1.0% or less within the specified pressure value of the present invention.

Even in the case where the silicone-treated filter was used, the CV value became large when the pressure value was out of the range of the specified pressure value.

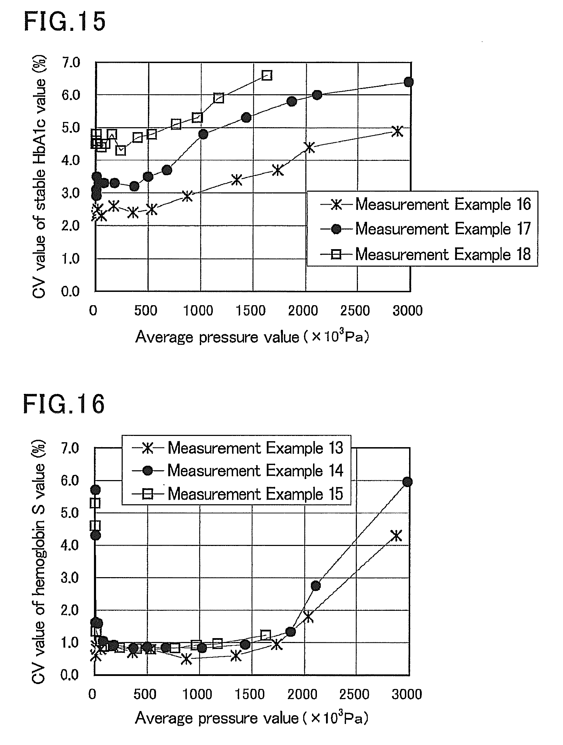

The relation between the average pressure value and the CV value of the stable hemoglobin A1c value at the time of measurement in the cases of Measurement Examples 16 to 18 where the silicone-untreated filter was used is shown in Fig. 15.

Reproducibility was unfavorable as the CV value was not 1.0% or less.

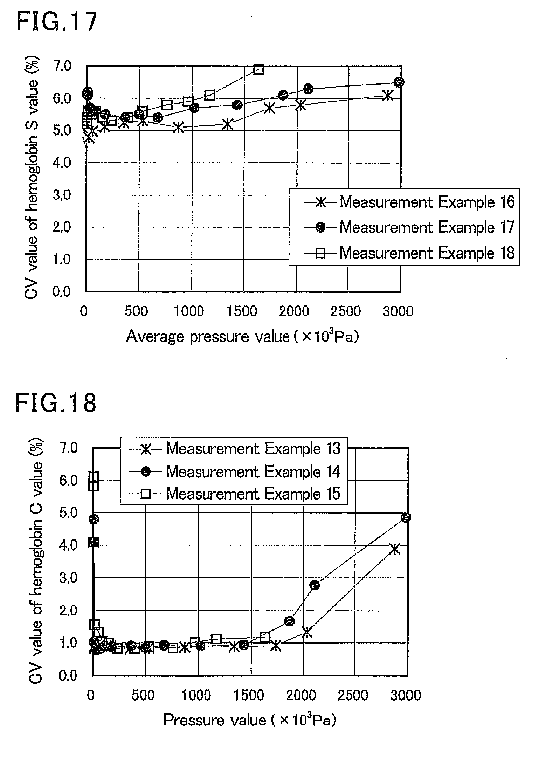

Similarly, the shift of the CV value of the hemoglobin S value in the cases of Measurement Examples 13 to 15 where the silicone-treated filter was used is shown in Fig. 16, and the shift of the CV value of the hemoglobin S value in the cases of Measurement Examples 16 to 18 where the silicone-untreated filter was used is shown in Fig. 17.

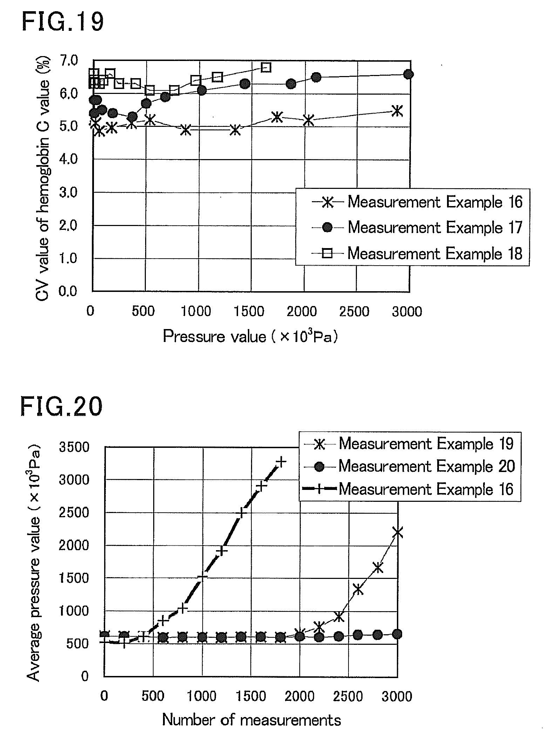

Moreover, the shift of the CV value of the hemoglobin C value in the cases of Measurement Examples 13 to 15 where the silicone-treated filter was used is shown in Fig. 18, and the shift of the CV value of the hemoglobin C value in the cases of Measurement Examples 16 to 18 where the silicone-untreated filter was used is shown in Fig. 19.

In the measurement of Measurement Examples 13 to 15 where the silicone-treated filter was used and the pressure value was within the range of the specified pressure value of the present invention, favorable CV values were obtained, and the CV value became large when the pressure value was out of the range of the specified pressure value even in the case where the silicone-treated filter was used.

Moreover, in the cases of Measurement Examples 16 to 18 where the silicone-untreated filter was used, reproducibility was unfavorable.

It was confirmed from the above results that the pressure value was stable and the reproducibility of measurement of the stable hemoglobin A1c value and the abnormal hemoglobin value was improved in the case where the silicone-treated filter was used and the pressure value was made within the specified pressure value of the present invention.

In addition, the similar behavior difference was shown when the silicone-treated column and the column not treated with silicone were compared.

### Evaluation (11) Evaluation of Effect of Repeated Measurement 1

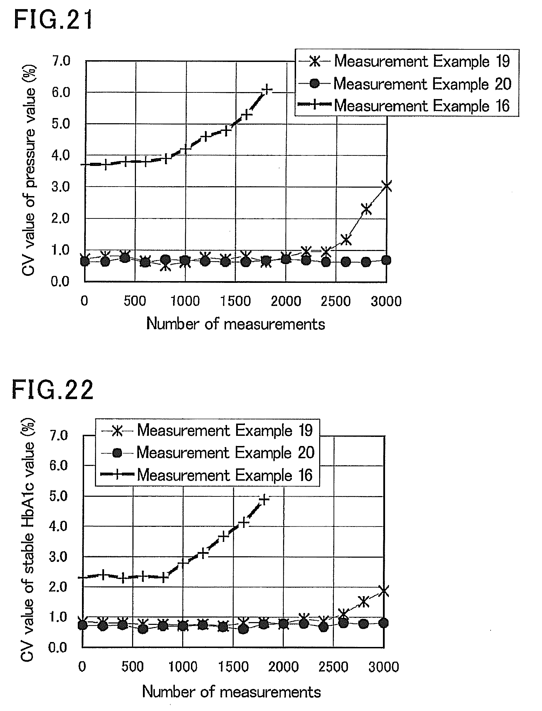

The shift of the reproducibility of the pressure value and the stable hemoglobin A1c value when a sample of the healthy human blood was measured 3000 times in total under the measurement conditions described in Table 4 was confirmed by Measurement Examples 16, 19, and 20 in Table 6. The shift of the average pressure value, the shift of the CV value of the pressure value, and the shift of the CV value of the stable hemoglobin A1c value at the time of carrying out the "(8) Reproducibility Test of Stable Hemoglobin A1c Value" (n = 20) at every 200 measurement are shown in Fig. 20, Fig. 21, and Fig. 22, respectively.

In the case of Measurement Example 16 where the filter not treated with silicone was used, the average pressure value began to rise from about 500th measurement, on the other hand, the average pressure value of Measurement Example 19 in which the silicone-treated filter was used was stable until about 2000th measurement, and the average pressure value of Measurement Example 20 in which the silicone-treated filter and the silicone-treated column were used was stable until 3000th measurement.

Moreover, the CV value of the pressure value and the CV value of the stable hemoglobin A1c value were small and stable in Measurement Examples 19 and 20. Particularly, Measurement Example 20 showed favorable CV values even at 3000th measurement.

It was confirmed that from the above results the rise and the increase in variation of the pressure value were suppressed by the silicone-treated filter and the silicone-treated column and, as a result thereof, favorable reproducibility of the stable hemoglobin A1c value was able to be maintained.

### Evaluation (12) Evaluation of Effect of Repeated Measurement 2

The shift of the simultaneous reproducibility when a sample containing abnormal hemoglobin (AFSC hemocontrol) was measured 3000 times in total under the measurement conditions described in Table 5 was confirmed by Measurement Examples 16, 19, and 20 in Table 7.

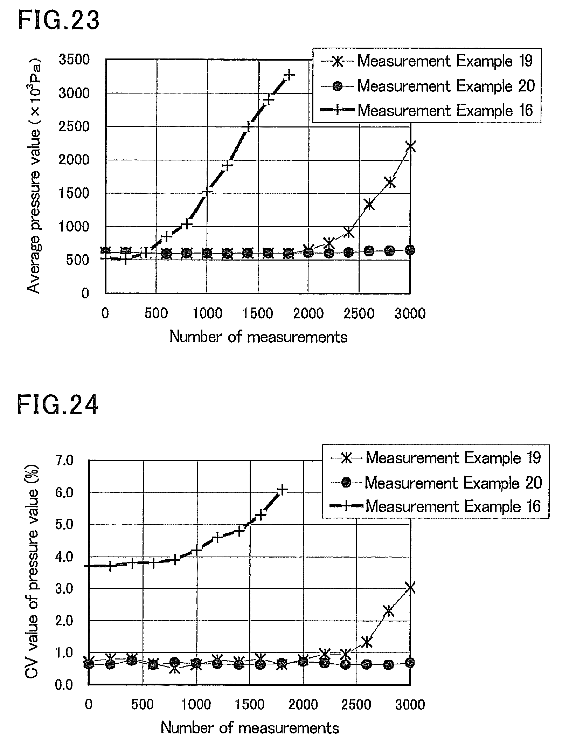

The shift of the average pressure value, the shift of the CV value of the pressure value, and the shift of the CV value of the stable hemoglobin A1c value at the time of carrying out the "(10) Reproducibility Test of Stable Hemoglobin A1c Value and Abnormal Hemoglobin Value" (n = 20) at every 200 measurement are shown in Fig. 23, Fig. 24, and Fig. 25, respectively.

In the case of Measurement Example 16 where the filter not treated with silicone was used, the average pressure value began to rise from about 500th measurement, on the other hand, the average pressure value of Measurement Example 19 in which the silicone-treated filter was used was stable until about 2000th measurement, and the average pressure value of Measurement Example 20 in which the silicone-treated filter and the silicone-treated column were used was stable until 3000th measurement.

Moreover, the CV value of the pressure value and the CV value of the stable hemoglobin A1c value were small and stable in Measurement Examples 19 and 20.

Particularly, Measurement Example 20 showed favorable CV values even at 3000th measurement.

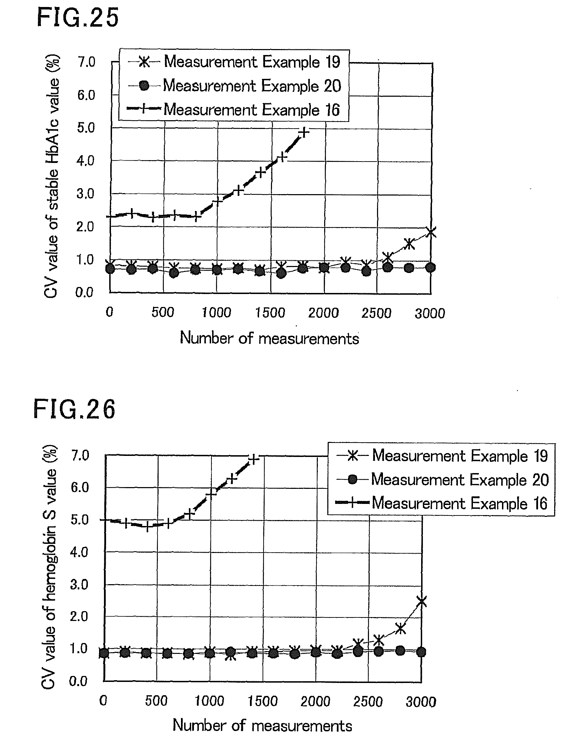

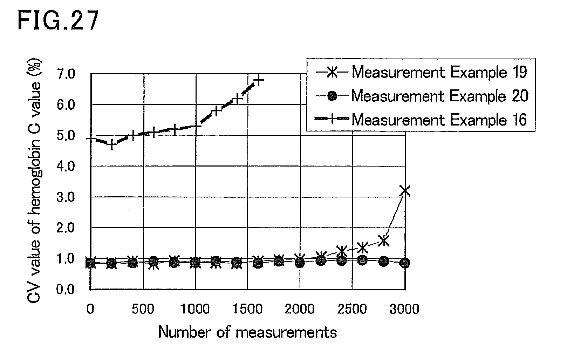

Moreover, the shift of the CV value of the hemoglobin S value is shown in Fig. 26, and the shift of the CV value of the hemoglobin C value is shown in Fig. 27.

The shifts of the CV values of these abnormal hemoglobins showed a shift similar to the shift of the CV value of the stable hemoglobin A1c value.

It was confirmed from the above results that the rise and the increase in variation of the pressure value were suppressed by the silicone-treated filter and the silicone-treated column and, as a result thereof, favorable reproducibility of the stable hemoglobin A1c value and the abnormal hemoglobin value was able to be maintained.

### INDUSTRIAL APPLICABILITY

According to the present invention, a method capable of measuring stable hemoglobin A1c using liquid chromatography in a short time with good reproducibility can be provided. Moreover, according to the present invention, a method for simultaneous measurement of stable hemoglobin A1c and abnormal hemoglobin using liquid chromatography can be provided.

### REFERENCE SIGNS LIST

- 1: Liquid chromatograph
- 2: Eluent switching valve
- 3: Liquid feeding pump
- 4: Pressure gauge
- 5: Injection valve
- 6: Pre-filter
- 7: Separation column
- 8: Detector
- 9: Eluent
- 10: Autosampler
- 11: Waste liquid container
- 21: Hemoglobin F (fetal hemoglobin)
- 22: Stable hemoglobin A1c
- 23: Hemoglobin A0
- 24: Hemoglobin S
- 25: Hemoglobin C
- 26: Hemoglobin A2

## Claims

1. A method for measuring stable hemoglobin A1c using liquid chromatography, comprising
installing on a flow path of a liquid chromatograph a filter whose surface is treated with a solution containing 1 to 50% by weight of a silicone oil or a solution containing 1 to 50% by weight of a silicone resin; and
setting a pressure value generated in a measurement system of the liquid chromatograph to 9.8 × 10³ Pa or more and 19.6 × 10⁵ Pa or less.

2. A method for measuring stable hemoglobin A1c using liquid chromatography, comprising
installing on a flow path of a liquid chromatograph a column main body whose inner surface is treated with a solution containing 1 to 50% by weight of a silicone oil or a solution containing 1 to 50% by weight of a silicone resin; and
setting a pressure value generated in a measurement system of the liquid chromatograph to 9.8 × 10³ Pa or more and 19.6 × 10⁵ Pa or less.

3. A method for measuring stable hemoglobin A1c using liquid chromatography, comprising
installing on a flow path of a liquid chromatograph
a filter whose surface is treated with a solution containing 1 to 50% by weight of a silicone oil or a solution containing 1 to 50% by weight of a silicone resin, and
a column main body whose inner surface is treated with a solution containing 1 to 50% by weight of a silicone oil or a solution containing 1 to 50% by weight of a silicone resin; and
setting a pressure value generated in a measurement system of the liquid chromatograph to 9.8 × 10³ Pa or more and 19.6 × 10⁵ Pa or less.

4. A method for simultaneous measurement of stable hemoglobin A1c and abnormal hemoglobin using liquid chromatography, comprising
installing on a flow path of a liquid chromatograph a filter whose surface is treated with a solution containing 1 to 50% by weight of a silicone oil or a solution containing 1 to 50% by weight of a silicone resin; and
setting a pressure value generated in a measurement system of the liquid chromatograph to 9.8 × 10³ Pa or more and 19.6 × 10⁵ Pa or less.

5. A method for simultaneous measurement of stable hemoglobin A1c and abnormal hemoglobin using liquid chromatography, comprising
installing on a flow path of a liquid chromatograph a column main body whose inner surface is treated with a solution containing 1 to 50% by weight of a silicone oil or a solution containing 1 to 50% by weight of a silicone resin; and
setting a pressure value generated in a measurement system of the liquid chromatograph to 9.8 × 10³ Pa or more and 19.6 × 10⁵ Pa or less.

6. A method for simultaneous measurement of stable hemoglobin A1c and abnormal hemoglobin using liquid chromatography, comprising
installing on a flow path of a liquid chromatograph
a filter whose surface is treated with a solution containing 1 to 50% by weight of a silicone oil or a solution containing 1 to 50% by weight of a silicone resin; and
a column main body whose inner surface is treated with a solution containing 1 to 50% by weight of a silicone oil or a solution containing 1 to 50% by weight of a silicone resin, and
setting a pressure value generated in a measurement system of the liquid chromatograph to 9.8 × 10³ Pa or more and 19.6 × 10⁵ Pa or less.
